# EUROPEAN PATENT APPLICATION

(11) **EP 2 687 234 A1**
(43) Date of publication of application: **22.01.2014**
(21) Application number: 12759886.0
(22) Date of filing: 15.03.2012
(51) Int. Cl.: A61K 49/00, A61K 49/04

(54) **SILICA NANOPARTICLES FOR DIAGNOSTIC IMAGING, METHOD FOR MANUFACTURING SAME, AND LABELING AGENT FOR BIOSUBSTANCE**

(30) Priority: 18.03.2011 JP 2011060739
(71) Applicant: Konica Minolta, Inc., Tokyo 100-7015 (JP)
(72) Inventor: AIMIYA, Takuji, Chiyoda-ku, Tokyo 100-7015 (JP); FURUSAWA, Naoko, Chiyoda-ku, Tokyo 100-7015 (JP); NAKANO, Yasushi, Chiyoda-ku, Tokyo 100-7015 (JP)
(74) Representative: Gille Hrabal
(86) International application number: PCT/JP2012/056623
(87) International publication number: WO 2012/128162

(57) **Abstract**

The present invention provides a silica nanoparticle for diagnostic imaging that is capable of use for X-ray CT, fluorescent imaging, or the like, and have high emission intensity. The present invention also provides a method for producing such a silica nanoparticle for diagnostic imaging. Also, a biosubstance labeling agent using the silica nanoparticle for diagnostic imaging is provided. The silica nanoparticle for diagnostic imaging of the present invention encapsulates an organic fluorescent dye and a metal complex, wherein a ligand of the metal complex form a covalent bond with constituent molecules of the silica nanoparticle.

## Description

### TECHNICAL FIELD

The present invention relates to a silica nanoparticle for diagnostic imaging that is capable of use in X-ray CT, fluorescent imaging, and the like, wherein the silica nanoparticle encapsulates an organic fluorescent dye and metal complex. The present invention also relates to a method for manufacturing the silica nanoparticle for diagnostic imaging and to a biosubstance labeling agent using the silica nanoparticle for diagnostic imaging.

### BACKGROUND ART

The diagnosis and treatment of cancer is performed by diagnostic imaging, followed by tissue diagnosis. After discovery of a pathological abnormality by X-ray computed tomography (abbreviated below as "X-ray CT") or by magnetic resonance imaging (abbreviated below as "MRI"), tissue of the pathological abnormality is surgically sampled while checking the pathological abnormality using an ultrasonic diagnostic equipment or the like. The X-ray CT or MRI and the ultrasonic diagnostic equipment differ in modality, so the range of excision is sometimes difficult to determine during this procedure.

That is to say, if a pathological abnormality which is detected by a method can be simultaneously observed in an imaging which can be used in a biopsy, it is thought that the accuracy of biopsies would be greatly improved, and it would become possible to avoid erroneous diagnoses that result from false negatives.

X-ray CT or MRI is capable of giving anatomical information. X-ray CT is based on detection of differences in X-ray absorption between normal tissue and tumor tissue. Molecules including an iodine atom, a gold atom, a gadolinium atom, and the like are used as a labeling agent for X-ray CT.

On the other hand, MRI is based on detection of differences of T₁ (spin-lattice relaxation time) and T₂ (spin-spin relaxation time) of tissue adjacent to an MRI contrast agent. Paramagnetic ions, including a gadolinium ion, are used as the MRI contrast agent. For example, Patent Document 1 discloses an MRI contrast agent having a gadolinium complex bonded to a surface of silica nanoparticles.

Since patients are not subjected to ionizing radiation, fluorescent imaging is always highly accepted as a diagnostic modality. Fluorescent imaging is based on detection of differences in the fluorescence emitted from normal tissue and tumor tissue. Organic fluorescent dyes that emit fluorescence at a wavelength different from that of an excitation light are excellent as fluorescent labeling agents.

Although a few biosubstance labeling agents have been known which can be used for both X-ray CT or MRI and fluorescent imaging so far, an organic dye is used as a fluorescent imaging component in such Biosubstance labeling agents (e.g., see Patent Document 2). However, in order to detect minute pathological abnormalities at an even earlier stage, fluorescence intensity of an organic dye alone is weak, and fluorescent substances are needed which have higher fluorescence intensity.

### CITATION LIST

### Patent Documents

Patent Document 1: Japanese Unexamined Patent Application Publication No. 2009-514905
Patent Document 2: Japanese Unexamined Patent Application Publication No. 2006-511473
Patent Document 3: WO2007/074722

### SUMMARY OF INVENTION

### Problem to be Solved by the Invention

The present invention was made in consideration of the aforementioned problems and circumstances. A problem to be solved by the present invention is to provide a silica nanoparticle for diagnostic imaging that may be used in X-ray CT, fluorescent imaging, and the like and that has a high emission intensity, and to provide the method for producing the silica nanoparticle. Moreover, another problem to be solved by the present invention is to provide a biosubstance labeling agent that is prepared by use of such a silica nanoparticle for diagnostic imaging.

### Solution to Problem

The problem of the present invention described above is solved by the means described below.

(1) A silica nanoparticle for diagnostic imaging encapsulating an organic fluorescent dye and a metal complex;
   wherein
   the organic fluorescent dye is coupled through an organic molecule to a framework of the silica nanoparticle; and
   the metal complex is coupled through an organic molecule to the framework of the silica nanoparticle.

(2) The silica nanoparticle for diagnostic imaging according to (1);
   wherein the organic molecule is an organic molecule comprising a silyl group.

(3) The silica nanoparticle for diagnostic imaging according to (1) or (2);
   wherein the organic molecule comprises, within a single molecule,
   an amino group, and
   a silyl group that is substituted by a hydrolyzable substituent group.

(4) The silica nanoparticle for diagnostic imaging according to any one of (1) to (3);
   wherein the organic molecule and a ligand of the metal complex are coupled through a covalent bond.

(5) The silica nanoparticle for diagnostic imaging according to (4);
wherein the covalent bond comprises an amide bond.

(6) The silica nanoparticle for diagnostic imaging according to any one of (1) to (5);
wherein the metal complex is a gadolinium complex.

(7) A method for producing a silica nanoparticle for diagnostic imaging;
   the method producing a silica nanoparticle for diagnostic imaging encapsulating an organic fluorescent dye and a metal complex, at least by the below listed step (a), step (b), and step (c):
   step (a): reacting an organic molecule comprising, within a single molecule, an amino group and a silyl group substituted by a hydrolyzable substituent group, and an organic fluorescent dye comprising a functional group that reacts with the amino group;
   step (b): reacting an organic molecule comprising, within a single molecule, an amino group and a silyl group substituted by a hydrolyzable substituent group, and a gadolinium complex comprising a functional group that reacts with the amino group; and
   step (c): mixing a silicon-containing alkoxide with the reaction product obtained by the step (a) and the reaction product obtained by the step (b), and performing a hydrolysis reaction under basic conditions.

(8) A biosubstance labeling agent;
wherein the silica nanoparticle for diagnostic imaging according to any one of claims 1 to 6 and a molecule labeling agent are bonded together through an organic molecule.

### ADVANTAGEOUS EFFECTS OF INVENTION

By the aforementioned means, the present invention is able to provide a silica nanoparticle for diagnostic imaging that is capable of use in X-ray CT, fluorescent imaging, and the like and that has high emission intensity, and the present invention is able to provide a method for producing such a silica nanoparticle for diagnostic imaging. Moreover, the present invention is able to provide a biosubstance labeling agent that is prepared by use of the silica nanoparticle for diagnostic imaging.

### DESCRIPTION OF EMBODIMENTS

The silica nanoparticle for diagnostic imaging of the present invention is a silica nanoparticle for diagnostic imaging that encapsulates an organic fluorescent dye and a metal complex, wherein the organic fluorescent dye is coupled through an organic molecule to a framework of the silica nanoparticle, and the metal complex is coupled through an organic molecule to the framework of the silica nanoparticle. This is a technical feature shared by the inventions according to claim 1 through claim 8.

In an embodiment of the present invention, the organic molecule preferably is an organic molecule having a silyl group, from the standpoint of realization of the effect of the present invention. The above organic molecule is further preferably a molecule having , within a single molecule, an amino group and a silyl group substituted by a hydrolyzable substituent group.

In the present invention, the organic molecule and the ligand of the metal complex are preferably coupled through a covalent bond. Further, the covalent bond preferably includes an amide bond. Further, the metal complex is preferably a gadolinium complex.

The method of production of a silica nanoparticle for diagnostic imaging of the present invention is preferably an embodiment that produces the silica nanoparticle for diagnostic imaging encapsulating an organic fluorescent dye and a metal complex through at least the aforementioned steps (a) to (c).

The silica nanoparticle for diagnostic imaging of the present invention may be suitably used for an embodiment of a biosubstance labeling agent wherein the silica nanoparticle for diagnostic imaging and a molecule labeling agent are coupled together through an organic molecule.

The present invention, constituent elements thereof, and aspects or embodiments for implementation of the present invention will be explained below in detail. In the present specification, the word "to" between a lower limit value and an upper limit value is taken to mean that the lower limit value and the upper limit value are included in the range.

### [General Description of the Silica Nanoparticle for Diagnostic Imaging]

The silica nanoparticle for diagnostic imaging of the present invention encapsulates an organic fluorescent dye and a metal complex, wherein the organic fluorescent dye and the metal complex are each coupled to the framework of the silica nanoparticle through an organic molecule.

In the present specification, the term "organic molecule" is taken to mean an organic molecule that has multiple sites capable of bonding with other substances, and particularly means an organic molecule that acts as a linker.

Further, the expression "framework of the silica nanoparticle" is taken to mean a backbone structure composed of bonds between oxygen atoms and silicon atoms composing a nano-sized (i.e. average particle diameter of 30 to 800 nm) silicon dioxide crystal.

The constituent elements of the silica nanoparticle for diagnostic imaging will be explained below in detail.

### [Encapsulation of Organic Fluorescent Dye]

The silica nanoparticle for diagnostic imaging of the present invention encapsulates an organic fluorescent dye, wherein the organic fluorescent dye is coupled to the framework of the silica nanoparticle through an organic molecule.

Methods for encapsulating the organic fluorescent dye by the silica nanoparticle include bonding between the organic molecule and the organic fluorescent dye in the silica nanoparticle by covalent bonding, ionic bonding, hydrogen bonding, or the like.

From the standpoint of chemical stability, the organic molecule and the organic fluorescent dye are preferably covalently bonded in the present invention. If the organic molecule and the organic fluorescent dye are not bonded together, the organic fluorescent dye may gradually leak while stored as an aqueous dispersion, and such leakage would be undesirable for use in a biosubstance labeling agent.

The organic fluorescent dye used for the silica nanoparticle for diagnostic imaging of the present invention is preferably an organic fluorescent dye that emits light in the visible to near infrared light of wavelength in the range of 400 to 900 nm when excited by ultraviolet to near infrared light of wavelength in the range of 200 to 700 nm.

The organic fluorescent dyes include fluorescein type dye molecules, rhodamine type dye molecules, Alexa Fluor (produced by Invitrogen Corp.) type dye molecules, BODIPY (produced by Invitrogen Corp.) type dye molecules, cascade type dye molecules, coumarin type dye molecules, eosin type dye molecules, NBD type dye molecules, pyrene type dye molecules, Texas Red type dye molecules, cyanine type dye molecules, and the like.

Specific examples of the organic fluorescent dye include 5-carboxy-fluorescein, 6-carboxy-fluorescein, 5,6-dicarboxy-fluorescein, 6-carboxy-2',4,4',5',7,7-hexachlorofluorescein, 6-carboxy-2',4,7,7'-tetrachlorofluorescein, 6-carboxy-4',5'-dichloro-2',7'-dimethoxyfluorescein, naphthofluorescein, 5-carboxy-rhodamine, 6-carboxy-rhodamine, 5,6-dicarboxy-rhodamine, rhodamine 6G, tetramethyl rhodamine, X-rhodamine, and Alexa Fluor 350, Alexa Fluor 405, Alexa Fluor 430, Alexa Fluor 488, Alexa Fluor 500, Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 635, Alexa Fluor 647, Alexa Fluor 660, Alexa Fluor 680, Alexa Fluor 700, Alexa Fluor 750, BODIPY FL, BODIPYTMR, BODIPY 493/503, BODIPY 530/550, BODIPY 558/568, BODIPY 564/570, BODIPY 576/589, BODIPY 581/591, BODIPY 630/650, BODIPY 650/665 (the above produced by Invitrogen Corp.), methoxycoumarin, eosin, NBD, pyrene, Cy 5, Cy 5.5, Cy 7, or the like. Such organic fluorescent dyes may be used singly or in combination of multiple types.

Various types of organic compounds that have a silyl group may be used as the organic molecule for encapsulating the organic fluorescent dye by the silica nanoparticle. Examples of such organic compounds that can be used include organic molecules that have a reactive functional group and at least one type of silyl group substituted by a hydrolyzable substituent group, i.e. an organic molecule that has, within a single molecule, a reactive substituent group and at least one type of silyl group that is substituted by hydrolyzable substituent group.

Here, the expression "silyl group" is a group that is provided for bonding with the "framework of the silica nanoparticle", described below and, in the present invention, a silyl group that has been substituted by a hydrolyzable substituent group is preferably used as this silyl group. The expression "silyl group substituted by a hydrolyzable substituent group" means a silyl group that has a substituent group such a group that hydrolyzes and generates a silanol group due to the presence of moisture. Examples of such silyl groups substituted by a hydrolyzable substituent group include alkoxysilyl groups such as the trimethoxysilyl group, triethoxysilyl group, and methyldimethoxysilyl group. The use of the "silyl group substituted by a hydrolyzable substituent group)" as the silyl group is advantageous in that it makes it possible to readily form a bond between the organic molecule and the "framework of the silica nanoparticle", described below and it makes it easy to control reactions of forming such a bond.

Thus no particular limitation is placed on the method of encapsulating the organic fluorescent dye by the silica nanoparticle, i.e. the method of bonding the organic fluorescent dye to the "framework of the silica nanoparticle" described below. However, as a preferred method, there can be used a reaction between the organic molecule that has the silyl group substituted by a hydrolyzable substituent group and the organic fluorescent dye that has a functional group capable of reacting with a reactive functional group. More specifically, this reaction can be performed as a reaction between the organic molecule that has a reactive functional group and at least one type of silyl group substituted by a hydrolyzable substituent group, and an organic fluorescent dye that has a functional group capable of reacting with the reactive functional group.

On the other hand, the expression "reactive functional group)" in the present specification refers to a functional group that is capable of forming a bond with another substance. This "reactive functional group" is normally a functional group that is different from the aforementioned "silyl group" and is capable of forming a bond with another substance.

The reactive functional group in the present invention that constitutes the "organic molecule for encapsulating the organic fluorescent dye by the silica nanoparticle", under conditions in which the ability of the aforementioned "silyl group" to bond with the below described "framework of the silica nanoparticles" is not lost, is preferably capable of bonding with the organic fluorescent dye, and particularly preferably is capable of bonding with the organic fluorescent dye without use of conditions for generating hydrolysis. Such reactive functional groups include an amino group, a mercapto group, a maleimide group, an isocyanate group, an isothiocyanate group, a carboxyl group, and an active ester group such as an N-hydroxysuccinimide group. Among these reactive functional groups, an amino group is preferred for stability and reactivity.

No particular limitation is placed on the molecules that have an amino group and have at least one type of silyl group substituted by a hydrolyzable substituent group. However, such molecules generally include various types of compounds which are called silane coupling agents. Specific examples of such compounds include 3-aminopropylmethyl dimethoxysilane, 3-aminopropyltriethoxysilane, 3-aminopropyltrimethoxysilane, or the like.

On the other hand, the organic fluorescent dyes that have the functional group capable of reacting with the reactive functional group include organic fluorescent dyes that have an amino group, a mercapto group, a maleimide group, an isocyanate group, an isothiocyanate group, a carboxyl group, and an active ester group such as an N-hydroxysuccinimide ester group.

When an amino group is used for the reactive functional group, as described above, among such reactive groups, the organic fluorescent are preferably has an isocyanate group, an isothiocyanate group, a carboxyl group, or an active ester group such as an N-hydroxysuccinimide ester group, as a functional group capable of reaction with the amino group.

In the present specification, the expression "active ester group" refers to an ester group that has a highly acidic electron-attracting group on the alcohol side of the ester group and activates nucleophilic reaction, i.e. an ester group that has high reactivity. The "active ester group" is actually an ester group that has an electron-attracting group on the alcohol side of the ester group and that is more active than an alkyl ester. The active ester group is reactive to groups such as an amino group, a thiol group, a hydroxyl group, and the like.

In the present invention, the reaction between the organic molecule and the organic fluorescent dye may be performed based on inherent reactivities of the aforementioned "reactive functional group" and the "functional group capable of reacting with the reactive functional group." Alternatively as may be required, an additive, a catalyst, or the like may be used which promotes the reaction, in order to activate these groups. For example, if the organic molecule has an amino group as the "reactive functional group", and if the organic fluorescent dye has a carboxyl group as the "functional group capable of reacting with the reactive functional group", a condensation agent may be used such as a carbodiimide like EDC (1-ethyl-3-[3-dimethylaminopropyl] carbodiimide hydrochloride, produced by Pierce Co., Ltd.), or the like.

Moreover, an organic solvent used may be any organic solvent that is unreactive with the hydrolyzable group on the silyl group. Such organic solvents include tetrahydrofuran, dimethyl sulfoxide, dimethylformamide, and the like.

Although no particular limitation is placed on the reaction temperature, the reaction may be performed in the temperature range of -20°C to 50°C.

The reaction time is preferably greater than or equal to 1 hour and less than or equal to 50 hours. Use of a reaction time greater than or equal to 1 hour is preferred from the standpoints of completion of the reaction and improvement of yield. Use of a reaction time less than or equal to 50 hours is preferred from the standpoints of preventing excessive reaction and preventing the formation of insoluble substances.

After completion of the reaction, the reaction product may be used in the following step without purification. As may be required, purification may be performed by routine methods such as recrystallization, column chromatography, and the like.

### [Encapsulation of Metal Complex]

The silica nanoparticle for diagnostic imaging of the present invention encapsulates a metal complex, wherein the metal complex is coupled to the framework of the silica nanoparticle through the organic molecule.

Methods for encapsulating the metal complex by the silica nanoparticle include bonding between the organic molecule and the organic fluorescent dye in the silica nanoparticle by covalent bonding, ionic bonding, hydrogen bonding, or the like. That is to say, the bonds that couple the metal complex and the organic molecule in the silica nanoparticle for diagnostic imaging according to the present invention include a covalent bond, ionic bond, hydrogen bond, and the like.

When the metal complex is not covalently bonded to the organic molecule, the metal complex may gradually leak while stored as a dispersed aqueous solution, which situation may be undesirable for use in a biosubstance labeling agent. As such, the metal complex is preferably bonded to the organic molecule by covalent bonding in the present invention. In this case, the bonding between the metal complex and the organic molecule is normally performed in a manner that a ligand of the metal complex and the organic molecule are coupled together by a covalent bond.

The covalent bonds include a ketone bond, an ester bond, a thioester bond, an amide bond, an ether bond, an imino bond, and the like. From the standpoint of high hydrolytic stability or the like, this covalent bond is preferably an amide bond.

The metal complexes include, but not limited to, various types of metal complexes such as lanthanide complexes, iron complexes, gold complexes, and the like. However, lanthanide complexes that form paramagnetic ions capable of use in MRI are preferred, and among such complexes, gadolinium complexes have high X-ray absorptivity and are further preferred.

Organic molecules that may be used as organic molecules for encapsulating the metal complex by the silica nanoparticle include the organic molecules used as the "organic molecule for encapsulating the organic fluorescent dye by the silica nanoparticles". In other words, various types of organic compounds may be used that have a silyl group. For example, an organic molecule may be used that has a reactive functional group and at least one type of silyl group substituted by a hydrolyzable substituent group. In this context, the "silyl group substituted by a hydrolyzable substituent group" may be the same groups as the "silyl group substituted by a hydrolyzable substituent group" that can be used for the aforementioned "organic molecule for encapsulating the organic fluorescent dye by the silica nanoparticles".

Furthermore, the organic molecule for encapsulating the metal complex by the silica nanoparticle may be the same or different from the organic molecule for encapsulating the organic fluorescent dye by the silica nanoparticle.

No particular limitation is placed on methods of encapsulation by covalent bonding between the metal complex and the organic molecule, i.e. the method of bonding between the metal complex and the below described "framework of the silica nanoparticles". However, it is possible to use a reaction between the organic molecule that has a reactive functional group and at least one type of silyl group substituted by a hydrolyzable substituent group, and the metal complex that has the functional group capable of reacting with the reactive functional group as a preferable method.

The reactive functional groups composing the "organic molecules for encapsulating the metal complex by the silica nanoparticles" in the present invention are preferably capable of bonding with the metal complex under conditions in which the ability of the aforementioned "silyl group" to bond with the "framework of the silica nanoparticle" described below is not lost. Such reactive functional groups include an amino group, mercapto group, maleimide group, isocyanate group, isothiocyanate group, carboxyl group, and an active ester group such as N-hydroxysuccinimide group. Among these reactive functional groups, an amino group, which forms an amide bond, is preferred from the standpoints of stability and reactivity.

Although no particular limitation is placed on the molecules that have an amino group and at least one type of silyl group substituted by a hydrolyzable substituent group, such molecules include various types of compounds referred to as silane coupling agents. Specific examples include 3-aminopropylmethyl dimethoxysilane, 3-aminopropyltriethoxysilane, 3-aminopropyltrimethoxysilane, and the like.

On the other hand, the metal complex having the functional group capable of reacting with the reactive functional group include metal complexes having an amino group, a mercapto group, a maleimide group, an isocyanate group, an isothiocyanate group, a carboxyl group, and an active ester group such as an N-hydroxysuccinimide group. Among such metal complexes, when an amino group is used for the reactive functional group, as described above, the functional groups of the metal complex that are reactive with the amino group include an isocyanate group, an isothiocyanate group, a carboxyl group, an active ester group such as an N-hydroxysuccinimide ester group, and the like. Among these functional groups, a carboxyl group and an active ester group such as an N-hydroxysuccinimide ester group are suitable which form an amide group.

Specific examples of the complex that has a carboxyl group, i.e. metal complex that has a carboxyl group, include gadolinium 1,4,7,10-tetraazacyclododecane-1,4,7,10-tetraacetate complex (abbreviated hereinafter as "Gd-DOTA"), gadolinium diethylene triamine pentaacetate complex (abbreviated hereinafter as "Gd-DTPA"), and the like.

In the present invention, the reaction between the organic molecule and the metal complex may be performed based on inherent reactivities of the aforementioned "reactive functional group" and the "functional group capable of reacting with the reactive functional group." Alternatively as may be required, an additive, a catalyst, or the like may be used which promotes the reaction, in order to activate these groups. For example, if the organic molecule has an amino group as the "reactive functional group", and if the metal complex has a carboxyl group as the "functional group capable of reacting with the reactive functional group", a condensation agent may be used such as a carbodiimide like EDC (1-ethyl-3-[3-dimethylaminopropyl]carbodiimide hydrochloride, produced by Pierce Co., Ltd.), or the like.

Moreover, an organic solvent use may be any organic solvent that is unreactive with the hydrolyzable group on the silyl group. Such organic solvents include tetrahydrofuran, dimethyl sulfoxide, dimethylformamide, and the like.

Although no particular limitation is placed on the reaction temperature, the reaction may be performed in the temperature range of -20°C to 50°C.

The reaction time is preferably greater than or equal to 1 hour and less than or equal to 50 hours. Use of a reaction time greater than or equal to 1 hour is preferred from the standpoints of completion of the reaction. Use of a reaction time less than or equal to 50 hours is preferred from the standpoints of preventing excessive reaction and preventing the formation of insoluble substances.

After completion of the reaction, the reaction product may be used in the following step without purification. As may be required, purification may be performed by routine methods such as recrystallization, column chromatography, and the like.

### [Framework of Silica Nanoparticle and Method for Producing the Same, and Particle Diameter of the Silica Nanoparticle for Diagnostic Imaging]

The silica nanoparticle for diagnostic imaging of the present invention encapsulates an organic fluorescent dye and a metal complex, wherein the organic fluorescent dye is coupled through an organic molecule to a framework of the silica nanoparticle, and the metal complex is coupled through an organic molecule to the framework of the silica nanoparticle.

Here, the "framework of the silica nanoparticle" in the present invention has a role of supporting the structure of the silica nanoparticle for diagnostic imaging as its matrix part.

A silica nanoparticle that does not encapsulate an organic fluorescent dye or the like, i.e. the "framework of the silica nanoparticles" itself, may be produced by various types of previously known production methods. For example, the silica nanoparticle is preferably produced by the so-called "Stober method", in which method hydrolysis of a silicon-containing alkoxide compound such as tetraethoxysilane or the like is performed under basic conditions using ammonia water or the like, as mentioned in Journal of Colloid Science, vol. 26, page 62 (1968). Alternatively, the "framework of the silica nanoparticle" may be also advantageously formed by performing hydrolysis of such a silicon-containing alkoxide compound in the presence of the organic fluorescent dye and metal complex into which was introduced the organic molecule, in the manner described below in the section titled "Method for Producing Silica Nanoparticle for Diagnostic Imaging]". The use of this method is advantageous in that the formation of the "framework of the silica nanoparticles" and the encapsulation of the organic fluorescent dye and the metal complex by the "framework of the silica nanoparticle" may be simultaneously performed.

In the silica nanoparticle for diagnostic imaging of the present invention, the organic fluorescent dye and metal complex exist encapsulated in the interior or on the surface of the aforementioned "framework of the silica nanoparticles" through the organic molecule.

The particle diameter of the silica nanoparticle for diagnostic imaging of the present invention may be adjusted freely by using publicly known reaction conditions, such as the added amount of water, amount of ethanol, amount of base, or the like. The average particle diameter may be adjusted to about 30 to 800 nm. Moreover, the coefficient of variance, which indicates variance of particle diameter, may be made less than or equal to 20%.

For the present invention, average particle diameter was determined by measuring cross-sectional areas for a sufficient number of particles by taking electron microscope images by use of a scanning electron microscope (SEM) and defining as the particle diameter a diameter calculated in assumption that the measured area is that of a corresponding circle. For the present specification, an arithmetic average of the particle diameters for 1,000 particles was used as the average particle diameter. coefficient of variance was taken to be the value calculated using the particle diameter distribution of the 1,000 particles.

### [Method for Producing Silica Nanoparticle for Diagnostic Imaging]

The method for producing a silica nanoparticle for diagnostic imaging of the present invention is that producing a silica nanoparticle for diagnostic imaging encapsulating an organic fluorescent dye and a metal complex, at least by the below listed step (a), step (b), and step (c):
step (a): reacting an organic molecule including, within a single molecule, an amino group and a silyl group substituted by a hydrolyzable substituent group, and an organic fluorescent dye including a functional group that reacts with the amino group;
step (b): reacting an organic molecule including, within a single molecule, an amino group and a silyl group substituted by a hydrolyzable substituent group, and a gadolinium complex including a functional group that reacts with the amino group; and
step (c): mixing a silicon-containing alkoxide with the reaction product obtained by the step (a) and the reaction product obtained by the step (b), and performing a hydrolysis reaction under a basic condition.

Publicly known methods including that mentioned in a non-patent document (Langmuir, vol. 8, page 2921 (1992)), for example, may be referred to for a method to produce the silica nanoparticle for diagnostic imaging that encapsulates both the organic fluorescent dye and the metal complex of the present invention.

The method for producing the silica nanoparticle for diagnostic imaging of the present invention, more specifically, is preferably a method producing a silica nanoparticle for diagnostic imaging encapsulating the organic fluorescent dye and metal complex through the steps (1) to (5) listed below.

Step (1): reacting a molecule including, within a single molecule, an amino group and at least one silyl group substituted by a hydrolyzable substituent group, and an organic fluorescent dye including a functional group reactive with the amino group.

Step (2): reacting a molecule including, within a single molecule, an amino group and at least one silyl group substituted by a hydrolyzable substituent group, and a gadolinium complex including a functional group reactive with the amino group.

Step (3): mixing a silicon-containing alkoxide compound, such as tetraethoxysilane, with the reaction product obtained by step (1) and the reaction product obtained by step (2).

Step (4): mixing an organic solvent such as ethanol, water, and a base in, and allowing the reaction to proceed.

Step (5): collecting a silica nanoparticle for diagnostic imaging encapsulating the organic fluorescent dye and the metal complex generated from the reaction mixture, by filtration or centrifugal separation.

Step (1) corresponds to the aforementioned step (a). Step (2) corresponds to the aforementioned step (b). Steps (3) to (5) correspond to the aforementioned step (c).

Then, the silica nanoparticle for diagnostic imaging of the invention of the present application that is obtained by the above steps (1) to (5) may further undergo the step (6) listed below to give a biosubstance labeling agent.

Step (6): bonding a molecule labeling agent to the silica nanoparticle for diagnostic imaging encapsulating the organic fluorescent dye and the metal complex obtained in step (5) to give a biosubstance labeling agent.

Details of the biosubstance labeling agent and the production method thereof will be described below in the section titled "Biosubstance Labeling Agent".

The silicon-containing alkoxide compounds used in the step (3) include tetraalkoxysilanes such as tetraethoxysilane and tetramethoxysilane, and trialkoxysilanes such as methyltrimethoxysilane, methylethoxysilane, phenyltriethoxysilane, and the like. They also include silicon-containing alkoxide compounds having an organic functional group. Specific examples include mercaptopropyltriethoxysilane, aminopropyltriethoxysilane, and the like.

The silicon-containing alkoxide compounds may be used singly or in combination of two or more types.

No particular limitation is placed on the mixing ratio of the silicon-containing alkoxide compound and an organic fluorescent dye-bonded molecule obtained in the step (1). However, they are preferably mixed so that the concentration in the finally obtained silica nanoparticle is greater than or equal to 1 x 10⁻⁶mol/L and less than or equal to 1 × 10⁻² mol/L. Sufficient fluorescence is obtained when the concentration is greater than or equal to 1 × 10⁻⁶ mol/L. A concentration less than or equal to 1 × 10⁻² mol/L is desirable from the standpoint of uniform dispersion within the silica.

Although no particular limitation is placed on the mixing ratio of the silicon-containing alkoxide compound and a gadolinium complex-bonded molecule obtained in the step (2), they are preferably mixed so that the concentration in the finally obtained silica nanoparticle is greater than or equal to 1 × 10⁻⁶ mol/L and less than or equal to 1 × 10⁻² mol/L. Sufficient X-ray CT or TRI contrast is obtained when the concentration is greater than or equal to 1 × 10⁻⁶ mol/L. A concentration less than or equal to 1 × 10⁻² mol/L is desirable from the standpoint of uniform dispersion within the silica.

The organic solvent used in the step (4) may be any organic solvent normally used in hydrolysis reactions of a silicon-containing alkoxide compound. The organic solvents used include methanol, ethanol, tetrahydrofuran, dimethylformamide, dimethyl sulfoxide, and the like. The organic solvents may be used singly or in combination of two or more types.

The base used in the step (4) may be any base normally used in hydrolysis reactions of a silicon-containing alkoxide compound. The bases used include ammonia, sodium hydroxide, potassium hydroxide, each of which may be used as an aqueous solution thereof.

Feed molar ratios are listed below in the case tetraethoxysilane is used as the silicon-containing alkoxide compound, ethanol is used as the organic solvent, and aqueous ammonia is used as the base.

Based on 1 mole of tetraethoxysilane, and taking the number of moles of ethanol to be "a," the number of moles of water to be "r," and the number of moles of ammonia to be "b," the tetraethoxysilane, ethanol, and ammonia water are mixed such that: a is greater than or equal to 20 and less than or equal to 400, r is greater than or equal to 10 and less than or equal to 200, and b is greater than or equal to 10 and less than or equal to 40. Specifically, the conditions mentioned in Journal of Colloid Science, viol. 26, page 62 (1968) may be used.

The reaction temperature in the step (4) is preferably that of a normal hydrolysis reaction of a silicon-containing alkoxide compound, and a temperature may be used in the range of room temperature to 50°C.

The reaction time in the step (4) may be that used for a normal hydrolysis reaction of a silicon-containing alkoxide compound. This reaction time is preferably greater than or equal to 1 hour and less than or equal to 50 hours. Use of a reaction time greater than or equal to 1 hour is preferred from the standpoints of completion of the reaction. Use of a reaction time less than or equal to 50 hours is preferred from the standpoints of preventing excessive reaction and preventing the formation of insoluble substances.

The method of collecting the silica nanoparticle for diagnostic imaging encapsulating the organic fluorescent dye and the metal complex generated from the reaction mixture in the step (5) may be a method that is normally performed for the recovery of the nanoparticle, such as filtration, centrifugal separation, or the like. As may be required, the collected silica nanoparticle for diagnostic imaging encapsulating the organic fluorescent dye and the metal complex may be washed with an organic solvent or water in order to remove unreacted raw material or the like.

### [Biosubstance Labeling Agent]

Biosubstance labeling agent of the present invention includes the above-mentioned silica nanoparticle for diagnostic imaging that encapsulates the organic fluorescent dye and the metal complex modified by the organic molecules, and also includes a molecule labeling agent bonded to the silica nanoparticle for diagnostic imaging.

The biosubstance labeling agent of the present invention may be an embodiment in which the molecule labeling agent bonds directly to the silica nanoparticle for diagnostic imaging without the use of another intermediary molecule. However, the Biosubstance labeling agent of the present invention is preferably an embodiment in which the silica nanoparticle for diagnostic imaging and the molecule labeling agent are bonded together by an organic molecule. That is to say, in a preferred embodiment of the biosubstance labeling agent of the present invention, the silica nanoparticle for diagnostic imaging and the molecule labeling agent are bonded together through an organic molecule. No particular limitation is placed on the bondings of the embodiment, and the bondings include covalent bonding, ionic bonding, hydrogen bonding, coordination bonding, physical absorption, chemical absorption, and the like. From the standpoint of bond stability, strong bonding is preferred, such as covalent bonding or the like.

In the biosubstance labeling agent of the present invention, the molecule labeling agent and the organic molecule bonded to the silica nanoparticle for diagnostic imaging may be bonded together directly, or alternatively, may be bonded together through a second linker compound.

The biosubstance labeling agent of the present invention can become an indicator for a biosubstance due to the molecule labeling agent binding and/or reacting specifically with a target biosubstance That is to say, the biosubstance labeling agent of the present invention can be used with advantage for applications to label a biosubstance. The biosubstance labeling agent of the present invention is capable of labeling by both modalities, i.e. fluorescence and X-ray absorption. In particular, the Biosubstance labeling agent of the present invention makes possible highly sensitive detection by various types of assay methods, such as immunoassay and the like.

### Molecule Labeling Agent

No particular limitation is placed on the molecule labeling agent in the present invention, and this molecule labeling agent may be any substance capable of binding and/or reacting specifically with the biosubstance that is a labeling target. Examples of the molecule labeling agent capable of use in the present invention include nucleotide chains, proteins, antibodies, and the like.

### Organic Molecule Bonding Together Silica Nanoparticle for Diagnostic Imaging and the Molecule Labeling Agent

The organic molecule used to bond together the silica nanoparticle for diagnostic imaging and the molecule labeling agent in the present invention may be any organic molecule, without particular limitation, that has a site capable of directly or indirectly bonding with the molecule labeling agent and has a site capable of bonding with the silica nanoparticle for diagnostic imaging. However, organic molecules capable of bonding with the molecule labeling agent as well as a surface of the silica nanoparticle for diagnostic imaging encapsulating the organic fluorescent dye and the metal complex in the present invention include silane coupling agents, which are compounds used widely for bonding between inorganic substances and organic substances. This silane coupling agent is a compound that has, at one end of the molecule, an alkoxysilyl group for imparting a silanol group by hydrolysis, and has at the other end of the molecule a functional group such as a carboxyl group, an amino group, an epoxy group, an aldehyde group, or the like. The silane coupling agent bonds to an inorganic substance through the oxygen atom of the silanol group. Specific examples include mercaptopropyltriethoxysilane, glycidoxypropyltriethoxysilane, aminopropyltriethoxysilane, or the like.

If used as the biosubstance labeling agent described below, it is possible to use a silane coupling agent that has a polyethylene glycol chain (e.g. PEG-silane no. SIM 6492.7, produced by Gelest, Inc.) in order to suppress non-specific absorption to a biosubstance.

These silane coupling agents may be used in combination of two or more kinds thereof.

As may be required, a second linker compound may be further placed between the organic molecule and the molecular targeting substance as a bond between the molecular targeting substance and the silica nanoparticle for diagnostic imaging of the present invention. Such a second linker compound may function as a spacer for assuring a fixed distance between the molecular targeting substance and the silica nanoparticle for diagnostic imaging. Alternatively, the second linker compound may function as an adapter for bonding between the molecular targeting substance and the organic molecule introduced to the silica nanoparticle for diagnostic imaging.

No particular limitation is placed on such a second linker compound as long as the second linker compound has a site capable of bonding directly with the organic molecule introduced to the silica nanoparticle for diagnostic imaging and has a site capable of bonding with the molecular targeting substance.

For example, if the organic molecule introduced to the silica nanoparticle for diagnostic imaging has an amino group, examples of the site of the second linker compound that is capable of direct bonding with the organic molecule include a carboxyl group and the like; and examples of the site capable of bonding with the molecular targeting substance include functional groups which are capable of reacting selectively with a mercapto group, such as the maleimide group. Specific examples of this second linker compound include sulfo-SMCC (sulfosuccinimidyl 4-[N-maleimidomethyl]cyclohexane-1-carboxylate, produced by Pierce Co., Ltd.) or the like.

### Method for producing Biosubstance Labeling Agent

The biosubstance labeling agent of the present invention is obtained by bonding together the silica nanoparticle for diagnostic imaging and the molecule labeling agent through the organic molecule.

The method for producing the biosubstance labeling agent of the present invention may include a step for introducing an organic molecule to the silica nanoparticle for diagnostic imaging, and then a step for bonding together the molecule labeling agent and the organic molecule. Alternatively, the present method may include a step for modifying the molecule labeling agent with an organic molecule beforehand to give an organic molecule-modified molecule labeling agent, and then a step for introducing the organic molecule-modified molecule labeling agent to the silica nanoparticle for diagnostic imaging. However, from the standpoints of facile post-processing or the like, the biosubstance labeling agent of the present invention is preferably produced by undergoing a step of introducing an organic molecule to the silica nanoparticle for diagnostic imaging, and then undergoing a step of bonding together the molecule labeling agent with the organic molecule.

An example of preferred methods for producing the biosubstance labeling agent of the present invention specifically includes the following:
(i) firstly, a step of reacting
   a silica nanoparticle for diagnostic imaging encapsulating the organic fluorescent dye and the metal complex and
   a silane coupling agent having a suitable reactive functional group (e.g., an aminopropyl group)
   to introduce the reactive functional group to the silica nanoparticle for diagnostic imaging, to give a functional group-modified silica nanoparticle for diagnostic imaging; and
(ii) a step of reacting
   the functional group-modified silica nanoparticle for diagnostic imaging obtained by step (i) and
   a molecule labeling agent (e.g., an antibody) having a functional group (e.g., carboxyl group) capable of forming a bond with the reactive functional group
   to generate a bond between the functional group-modified silica nanoparticle for diagnostic imaging and the molecule labeling agent, thereby to give a biosubstance labeling agent.

Alternatively, instead of the aforementioned step (ii), a step composed of the following two steps may be performed:
(ii') a step of reacting
   the functional group-modified silica nanoparticle for diagnostic imaging obtained by the aforementioned step (i), and
   an aforementioned second linker compound having a functional group capable of forming a bond with the reactive functional group (e.g., a carboxyl group and active esters thereof) and a second reactive functional group (e.g. a maleimide group) (e.g., sulfo-SMMC)
   to introduce the second reactive functional group to the silica nanoparticle for diagnostic imaging, thereby to give a second functional group-modified silica nanoparticle for diagnostic imaging; and
(iii') a step of reacting
   the second functional group-modified silica nanoparticle for diagnostic imaging obtained by step (ii'), and
   a molecule labeling agent (e.g. an antibody) having a functional group that is capable of forming a bond with the second reactive functional group (e.g. a thiol group)
   to form a bond between this second functional group-modified silica nanoparticle for diagnostic imaging and the molecule labeling agent, thereby to give a biosubstance labeling agent.

Procedures for the reaction between the silane coupling agent and the silica nanoparticle for diagnostic imaging encapsulating the organic fluorescent dye and the metal complex may be those of widely known techniques. For example, the obtained silica nanoparticle for diagnostic imaging encapsulating the organic fluorescent dye and the metal complex is dispersed in purified water, aminopropyltriethoxysilane is added, and the resultant mixture is reacted for 12 hours at room temperature. After completion of the reaction, a silica nanoparticle for diagnostic imaging that is surface-modified by the aminopropyl group may be obtained by centrifugal separation or filtration.

Moreover, as a specific example of the step (ii), it is possible to react a carboxyl group within the antibody and an amino group of the silica nanoparticle for diagnostic imaging modified by aminopropyltriethoxysilane, and thus bond the antibody to the silica nanoparticle for diagnostic imaging through an amide bond. As may be required, it is possible to use a condensation agent such as EDC (1-ethyl-3-[3-dimethylaminopropyl]carbodiimide hydrochloride, produced by Pierce Co., Ltd.).

As mentioned in the steps (ii') and (iii'), as may be required, it is possible to use a linker compound having a site capable of bonding with the molecular targeting substance and a site capable of directly bonding with the organic molecule-modified silica nanoparticle for diagnostic imaging (i.e. the aforementioned second linker compound). As a specific example, it is possible to use sulfo-SMCC (sulfosuccinimidyl 4-[N-maleimidomethyl]-cyclohexane-1-carboxylate, produced by Pierce Co., Ltd.), which has both a site that reacts selectively with an amino group and a site that reacts selectively with a mercapto group, to bond together the amino group of the silica nanoparticle for diagnostic imaging modified by aminopropyltriethoxysilane and a mercapto group in the antibody to give a silica nanoparticle for diagnostic imaging bonded to the antibody.

### EXAMPLES

The present invention is described in detail below using examples, but the present invention is not limited to these examples. Furthermore, unless otherwise indicated, "parts" and "%" in the examples indicate "parts by mass" and "mass%," respectively.

Synthesis Example 1: Production of Silica Nanoparticles for Diagnostic Imaging 1 Encapsulating Organic Fluorescent Dye TAMRA and Gadolinium Complex Gd-DTPA

Silica nanoparticles for diagnostic imaging 1 were produced by a method including the steps (1) to (6) listed below.

Step (1) : 2.6mg (0.0048 mmol) of N-hydroxysuccinimide ester derivative of an organic fluorescent dye TAMRA (referred to below as "TAMRA") (produced by Invitrogen Corp., 5(6)-TAMRA-NHS, SE) was dissolved in 0.8 mL of dimethylformamide, and under ice cooling, 1 µL (0.0048 mmol) of 3-aminopropyltriethoxysilane (produced by Gelest, Inc.) was added to the mixture. Then a resultant mixture was stirred for 30 minutes.

Step (2): 2.6 mg (0.0048 mmol) of gadolinium diethylenetriamine pentaacetate complex (produced by Aldrich) was dissolved in 0.8 mL of dimethylformamide (abbreviated hereinafter as "DMF"). Them 0.7 mg (0.0048 mmol) of 1-ethyl-3-[3-dimethylaminopropyl] carbodiimide hydrochloride (produced by Pierce Co., Ltd.), and then 1 µL (0.0048 mmol) of 3-aminopropyltriethoxysilane (produced by Gelest, Inc.) were added, and the mixture was stirred for 30 minutes.

Step (3): The DMF solutions obtained in steps (1) and (2) and 40 µL of tetraethoxysilane were mixed together.

Step (4): 40 mL of ethanol and 10 mL of aqueous 14% ammonia were mixed together.

Step (5) : While stirring the mixed solution were produced in step (4) at room temperature, the mixed solution produced in step (3) was added. A resultant mixture was stirred for 12 hours after the start of addition.

Step (6): the reaction mixture was centrifuged at 10,000 g for 60 minutes, and a resultant supernatant was removed. Ethanol was added to allow the precipitate dispersed, and a centrifugal separation was performed again. An ethanol wash and a purified water wash were performed in the same procedure.

A scanning electron microscope (SEM, manufactured by Hitachi, model S-800) was used for observation of resultant silica nanoparticles for diagnostic imaging 1. According to the observation, average particle diameter was 110 nm, and the coefficient of variance was 12%. This observation ways performed by measuring cross-sectional areas of 1,000 particles, determining diameter corresponding to an area of a circle corresponding to measured value of the cross-sectional area as a particle diameter, and taking average particle diameter to be arithmetic average of the particle diameters of 1, 000 particles. The coefficient of variance was calculated from the particle diameter distribution of 1,000 particles, too.

Synthesis Example 2: Synthesis of Silica Nanoparticles for Diagnostic Imaging 2 Encapsulating Cy 5 and Gd-DOTA

Silica nanoparticles for diagnostic imaging 2 were synthesized by the same procedure as that described in the Synthesis Example 1 except that Cy 5 N-hydroxysuccinimide ester derivative (produced by GE Healthcare) was used as the organic fluorescent dye and using Gd-DOTA (produced by Guerbet, DOTAREM ®) was used as the gadolinium complex.

Resultant silica nanoparticles for diagnostic imaging 2 were observed by SEM. According to the observation, average particle diameter was 100 nm, and the coefficient of variance was 10%.

Synthesis Example 3: Synthesis of Silica Nanoparticles for Diagnostic Imaging 3 Encapsulating TAMRA/Gd-DTPA

Silica nanoparticles for diagnostic imaging 3 were produced by a method including the steps (1) to (6) listed below, without performing a step for covalent bonding the Gd-BTPA to the silica nanoparticles.

Step (1): First, 2.6 mg (0.0048 mmol) of N-hydroxysuccinimide ester derivative of TAMRA (produced by Invitrogen Corp., 5(6)-TAMRA-NHS, SE) was dissolved in 0.8 mL of dimethylformamide. Then under ice cooling, 1 µL (0.0048 mmol) of 3-aminopropyltriethoxysilane (produced by Gelest, Inc.) was added, and a resultant mixture was stirred for 30 minutes.

Step (2): 2.6 mg (0.0048 mmol) of gadolinium diethylenetriamine pentaacetate complex (produced by Aldrich) was dissolved in 0.8 mL of dimethylformamide.

Step (3): The DMF solutions obtained in steps (1) and (2) were mixed with 40 µL of tetraethoxysilane.

Step (4): 40 mL of ethanol and 10 mL of aqueous 14% ammonia were mixed.

Step (5): While stirring the mixed solution produced in step (4) at room temperature, the mixed solution produced in step (3) was added. Stirring was performed for 12 hours after the start of addition.

Step (6): 1 the reaction mixture was centrifuged at 10,000 g for 60 minutes, and a resultant supernatant was removed. Ethanol was added to allow the precipitate dispersed, and a centrifugal separation was performed again. An ethanol wash and a purified water wash were performed in the same procedure

A scanning electron microscope (SEM, manufactured by Hitachi, model S-800) was used for observation of resultant silica nanoparticles for diagnostic imaging 3. According to the observation, average particle diameter was 105 nm, and the coefficient of variance was 12%.

Synthesis Example 4: Synthesis of Silica Nanoparticles for Diagnostic Imaging 4 Encapsulating TAMRA

Silica nanoparticles for diagnostic imaging 4 encapsulating only the organic fluorescent dye were produced by a method including the steps (1) to (5) listed below.

Step (1): First, 2.6 mg (4) mmol) of N-hydroxysuccinimide ester derivative of TAMRA (produced by Invitrogen Corp., 5(6)-TAMRA-NHS, SE) was dissolved in 0. 8 mL of dimethylformamide. Then under ice cooling, 1 µL (0.0048 mmol) of 3-aminopropyltriethoxysilane (produced by Gelest, Inc.) was added, and a resultant mixture was stirred for 30 minutes.

Step (2): The DMF solution obtained in step (1) and 40 µL of tetraethoxysilane were mixed together.

Step (3): 40 mL of ethanol and 10 mL of aqueous 14% ammonia water were mixed together.

Step (4) : While stirring the mixed solution produced in step (4) at room temperature, the mixed solution produced in step (2) was added. A resultant mixture was stirred for 12 hours after the start of addition.

Step (5): the reaction mixture was centrifuged at 10,000 g for 60 minutes, and a resultant supernatant was removed. Ethanol was added to allow the precipitate dispersed, and a centrifugal separation performer again. An ethanol wash and a purified water wash were performed in the same procedure.

A scanning electron microscope (SEM, manufactured by Hitachi, model S-800) was used for observation of resultant silica nanoparticles for diagnostic imaging 4. According to the observation, average particle diameter was 110 nm, and the coefficient of variance was 10%.

### Synthesis Example 5: Synthesis of Silica Nanoparticles for Diagnostic Imaging 5 Encapsulating GD-DTPA

Silica nanoparticles for diagnostic imaging 5 encapsulating only the gadolinium complex covalently bonded to the silica particle were produced by a method including the steps (1) to (5) listed below.

Step (1): 2.6 mg (0.0048 mmol) of gadolinium diethylenetriamine pentaacetate complex (produced by Aldrich) was dissolved in 0.8 8 mL of dimethylformamide. Then 0.7 mg (0.0048 mmol) of 1-ethyl-3-[3-dimethylaminopropyl] carbodiimide hydrochloride (produced by Pierce Co., Ltd.), and then 1 µL (0.0048 mmol) of 3-aminopropyltriethoxysilane (produced by Gelest, Inc.) were added, and a resultant mixture was stirred for 30 minutes.

Step (2): The DMF solution obtained in step (1) and 40 µL of tetraethoxysilane were mixed together.

Step (3): 40 mL of ethanol and 10 mL of aqueous 14% ammonia water were mixed together.

Step (4) : While stirring the mixed solution produced in step (4) at room temperature, the mixed solution produced in step (2) was added. A resultant mixture was stirred for 12 hours after the start of addition.

Step (5): the reaction mixture was centrifuged at 10,000 g for 60 minutes, and a resultant supernatant was removed. Ethanol was added to allow the precipitate dispersed, and a centrifugal separation was performed again. An ethanol wash and a purified water wash was performed in the same procedure.

A scanning electron microscope (SEM, manufactured by Hitachi, model S-800) was used for observation of silica nanoparticles for diagnostic imaging 5. According to the observation, average particle diameter was 102 nm, and the coefficient of variance was 11%.

Respective 1 nM PBS (phosphate buffered saline) dispersions of the obtained silica nanoparticles were prepared, and a fluorescence spectrophotometer F-7000 (commercial product name, manufactured by Hitachi High-Technologies Corp.) was used to measure fluorescence intensity at an excitation light wavelength of 580 nm (for TAMRA encapsulation) or 633 nm (for Cy 5 encapsulation). Fluorescence intensity was evaluated as a relative value by normalization by taking fluorescence intensity at 580 nm for the produced silica nanoparticles for diagnostic imaging 1 to be 100. Evaluation results are shown in Table 1.

X-ray absorptivity was measured by placing a 1 nM PBS dispersion solution of the obtained silica nanoparticles in a plastic tube, irradiating the nanoparticles with X-rays at an acceleration voltage of 70 kV, and measuring the amount of transmitted X-rays using a flat panel detector PaxScan® 1313R (commercial product name, manufactured by Varian Medical Systems, Inc.). X-ray absorptivity was evaluated as a relative value by normalization by taking the measurement value of the part free of sample as 100. Evaluation results are shown in Table 1. In Table 1, lower values indicated in the "X-ray absorption" column indicate a smaller amount of transmitted X-rays and increasingly excellent X-ray absorptivity.

**Table 1**

| No. of silica nanoparticles for diagnostic imaging | Fluorescence | X-ray absorption | Remarks |
|---|---|---|---|
| 1 | 100 | 10 | Present Invention |
| 2 | 80 | 5 | Present invention |
| 3 | 95 | 90 | Comparative Examples |
| 4 | 98 | 92 | Comparative Examples |
| 5 | 0 | 10 | Comparative Examples |

As made clear by the results shown in Table 1, the silica nanoparticles for diagnostic imaging 1 and 2 of the present invention were found to be detected by both modalities of fluorescence and X-ray absorption. Moreover, X-ray absorptivity was deteriorated for the particles 3, which did not encapsulate by covalent bonding of the gadolinium complex. This deterioration is assumed to have been due to the gadolinium complex not being bonded to the framework of the particles by covalent bonding, so that gadolinium complex readily leaked from the silica particles, and the gadolinium complex was remove by washing. The particles 4, which encapsulated only an organic fluorescent dye, could not be detected by X-ray absorption, and detection was possible only by fluorescence. Moreover, the particles 5, which only encapsulated the gadolinium complex, could not be detected by fluorescence, and detection was possible only by X-ray absorption. Detection by multiple modalities was not possible for the particles that encapsulated only either of the organic fluorescent dye and the gadolinium complex, and the effect of the present invention was not obtained for such particles.

### ≪Biosubstance Labeling Agent≫

Molecule-modified silica nanoparticles A and B wore prepared by the method indicated below, by using the silica nanoparticles for diagnostic imaging 1 and 2, which encapsulated both an organic fluorescent dye and a metal complex, respectively. These molecule-modified silica nanoparticles were used to further prepare biosubstance labeling agents 1 and 2, and long-term storage ability of the biosubstance labeling agents was evaluated.

### [Preparation of Molecule-modified Silica Nanoparticles A]

### (Molecule-modified silica nanoparticles A: amino group modification of silica nanoparticles for diagnostic imaging encapsulating TAMRA/Gd-DTPA)

1 mg of silica nanoparticles for diagnostic imaging 1 was dispersed in 5 mL of purified water. Then 100 µL of an aqueous dispersion of aminopropyltriethoxysilane was added, and a resultant mixture was stirred for 12 hours at room temperature.

The reaction mixture was centrifuged at 10,000 g for 60 minutes, and a resultant supernatant was removed. Ethanol was added to allow the precipitate dispersed, and a centrifugal separation was performed again. An Ethanol wash and a purified water wash were performed in the same procedure. Molecule-modified silica nanoparticles A were thus obtained.

FT-IR measurement of the obtained amino group-modified silica nanoparticles A was performed. Absorption due to an amino group could be observed, and it was confirmed that the silica nanoparticles had been successfully modified with an amino group.

### [Preparation of Molecule-modified Silica Nanoparticles B]

### (Molecule-modified silica nanoparticles B: amino group modification of silica nanoparticles for diagnostic imaging encapsulating Cy 5/Gd-DOTA)

Amino group modification was performed for the silica nanoparticles for diagnostic imaging 2, in the same procedure as that used for the molecule-modified silica nanoparticles A. Molecule-modified silica nanoparticles B were thus obtained.

FT-IR measurement was performed for the obtained amino group-modified silica nanoparticles for diagnostic imaging that encapsulated silica-coated tetramethyl rhodamine, i.e. the molecule-modified silica nanoparticles B. Absorption due to an amino group could be observed, and it was confirmed that the silica nanoparticles had been successfully modified with an amino group.

### [Preparation of Biosubstance Labeling Agent 1]

### (Biosubstance labeling agent 1: Antibody conjugate of amino group-modified silica nanoparticles for diagnostic imaging encapsulating TAMRA/Gd-DTPA)

First, to 2 mL of DMSO was added 0.1 mL of a dispersion prepared by dispersing 0.5 mg of the amino group-modified silica nanoparticles for diagnostic imaging encapsulating TAMRA/Gd-DTPA obtained in the above "Preparation of Molecule-modified Silica Nanoparticles A," i.e. the molecule-modified silica nanoparticles A, in 0.5 mL of purified water. Then sulfo-SMCC (produced by Pierce Co., Ltd.) was added, and a resultant mixture was allowed to react for 1 hour. Excess sulfo-SMCC or the like was removed by centrifugal separation. Separately, anti-hCG antibody was reduced using 1M dithiothreitol (DTT), and excess DTT was removed using a gel filtration column.

The sulfo-SMCC-treated silica nanoparticles for diagnostic imaging encapsulating TAMRA/Gd-DTPA and the DTT-treated anti-hCG antibody were mixed, and a resultant mixture was allowed to react for 1 hour. Then 10 mM mercaptoethanol was added, and the reaction was stopped. A gel filtration column was used to remove unreacted material, to give anti-hCG antibody-bonded silica nanoparticles for diagnostic imaging encapsulating TAMRA/Gd-DTPA (biosubstance labeling agent 1).

### [Preparation of Biosubstance Labeling Agent 2]

### (Biosubstance labeling agent 2: Antibody conjugate of amino group-modified silica nanoparticles for diagnostic imaging encapsulating Cy 5/Gd-DOTA)

In the same procedure as that used to prepare the biosubstance labeling agent 1, the amino group-modified silica nanoparticles for diagnostic imaging encapsulating Cy 5/Gd-DOTA obtained by the aforementioned "Preparation of Molecule-modified Silica Nanoparticles B," i.e. the molecule-modified silica nanoparticles B, were used to obtain the anti-hCG antibody-bonded silica nanoparticles for diagnostic imaging encapsulating Cy 5/Gd-DOTA (biosubstance labeling agent 2).

Immunoassay was performed for the biosubstance labeling agents 1 and 2 in the procedure described below.
(1) The anti-hα subnit was fixed in wells on a microplate.
(2) The hCG which is the antigen was placed in each of the wells while the concentration of the hCG varied between wells.
(3) After washing to remove excess hCG, a dispersion of the biosubstance labeling agent was placed in each of the wells.
(4) Excess biosubstance labeling agent was removed by washing.
(5) Fluorescence intensity was measured in each well using a microplate reader Fluoroskan Ascent FL (commercial product name, manufactured by Thermo Fisher Scientific, Inc.).
(6) The microplate was irradiated with X-rays, and X-ray absorptivity was measured for each well.

The fluorescence intensities measured at each hCG antibody concentration are shown in Table 2, where the fluorescence intensity of biosubstance labeling agent 1 is taken to be 100 when the hCG antibody concentration was 1 ng/mL. The X-ray absorptivity values measured at each hCG antibody concentration are shown in Table 3, where the X-ray absorptivity of biosubstance labeling agent 1 is taken to be 10 when the hCG antibody concentration was 1 ng/mL.

**Table 2**

| hCG Antibody concentration | Biosubstance labeling agent 1 | Biosubstance labeling agent 2 |
|---|---|---|
| 0.01 ng/ml | 2 | 1 |
| 0.10 ng/ml | 15 | 10 |
| 1.00 ng/ml | 100 | 90 |
| 10.00 ng/ml | 950 | 910 |

**Table 3**

| hCG Antibody concentration | Biosubstance labeling agent 1 | Biosubstance labeling agent 2 |
|---|---|---|
| 0.01 ng/ml | 100 | 100 |
| 0.10 ng/ml | 90 | 93 |
| 1.00 ng/ml | 10 | 16 |
| 10.00 ng/ml | 2 | 3 |

When the biosubstance labeling agent of the present invention was used, a change was seen for both modalities of fluorescence and X-ray absorption, i.e. the fluorescence intensity increased and the X-ray absorption declined according to the concentration of the antigen.

That is to say, the biosubstance labeling agent obtained by the present invention does not detract from the ability to recognize the antigen. That is to say, based on this result, it is understood that the present invention can provide a biosubstance labeling agent that is capable of highly sensitive detection using multiple modalities.

## Claims

1. A silica nanoparticles for diagnostic imaging encapsulating an organic fluorescent dye and a metal complex;
wherein
the organic fluorescent dye is coupled through an organic molecule to a framework of the silica nanoparticle; and
the metal complex is coupled through an organic molecule to the framework of the silica nanoparticle.

2. The silica nanoparticle for diagnostic imaging according to claim 1;
wherein the organic molecule is an organic molecule comprising a silyl group.

3. The silica nanoparticle for diagnostic imaging according to claim 1 or 2;
wherein the organic molecule comprises, within a single molecule,
an amino group, and
a silyl group substituted by a hydrolyzable substituent group.

4. The silica nanoparticle for diagnostic imaging according to any one of claims 1 to 3;
wherein the organic molecule and a ligand of the metal complex are coupled through a covalent bond.

5. The silica nanoparticle for diagnostic imaging according to claims 4;
wherein the covalent bond comprises an amide bond.

6. The silica nanoparticles for diagnostic imaging according to any one of claims 1 to 5;
wherein the metal complex is a gadolinium complex.

7. A method for producing a silica nanoparticle for diagnostic imaging;
the method producing a silica nanoparticle for diagnostic imaging encapsulating an organic fluorescent dye and a metal complex, at least by the below listed step (a), step (b), and step (c):
step (a): reacting an organic molecule comprising, within a single molecule, an amino group and a silyl group substituted by a hydrolyzable substituent group, and an organic fluorescent dye comprising a functional group that reacts with the amino group;
step (b): reacting an organic molecule comprising, within a single molecule, an amino group and a silyl group substituted by a hydrolyzable substituent group, and a gadolinium complex comprising a functional group that reacts with the amino group; and
step (c): mixing a silicon-containing alkoxide with the reaction product obtained by the step (a) and the reaction product obtained by the step (b), and performing a hydrolysis reaction under basic conditions.

8. A biosubstance labeling agent;
wherein the silica nanoparticle for diagnostic imaging according to any one of claims 1 to 6 and a molecule labeling agent are bonded together through an organic molecule.
